(19) Europäisches Patentamt / European Patent Office / Office européen des brevets

(11) **EP 3 838 150 A1**

(12) **EUROPEAN PATENT APPLICATION**

(43) Date of publication:
23.06.2021 Bulletin 2021/25

(51) Int Cl.:
*A61B 5/361* (2021.01)     *A61B 5/00* (2006.01)
*A61B 5/364* (2021.01)     *A61B 5/024* (2006.01)

(21) Application number: 20212430.1

(22) Date of filing: 08.12.2020

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
KH MA MD TN

(30) Priority: 16.12.2019  JP 2019226464

(71) Applicant: UNION TOOL CO.
Tokyo 140-0013 (JP)

(72) Inventors:
• **Shinozaki, Ryo**
  **Tokyo, 140-0013 (JP)**
• **Matsui, Taishi**
  **Tokyo, 140-0013 (JP)**
• **Nakata, Akio**
  **Tokyo, 140-0013 (JP)**

(74) Representative: **Hoffmann Eitle**
**Patent- und Rechtsanwälte PartmbB**
**Arabellastraße 30**
**81925 München (DE)**

(54) **ATRIAL FIBRILLATION DETECTION SYSTEM**

(57)    An object of the present invention is to provide an atrial fibrillation detection system in which: the extent of irregularity in measured pulse intervals is calculated; even in cases where an extrasystole has occurred, detection of a false positive is reduced even when the extrasystole is not excluded; and highly reliable assessment results are obtained. An atrial fibrillation detection system comprising: pulse interval measurement means 4 that measures the pulse intervals of a heart; pulse interval conversion means 8 that performs conversion, using a prescribed function; entropy computation means 9 that calculates entropy S from pulse interval images r obtained through conversion by the pulse interval conversion means 8; and comparative assessment means 10 that compares the entropy S calculated by the entropy computation means 9 and a threshold value, and that, in cases where the entropy S is greater than the threshold value, assesses that atrial fibrillation is occurring.

FIG.1

1 ATRIAL FIBRILLATION DETECTION SYSTEM

| PULSE INTERVAL MEASUREMENT MEANS | 4 |
| PULSE INTERVAL CONVERSION MEANS | 8 |
| ENTROPY COMPUTATION MEANS | 9 |
| COMPARATIVE ASSESSMENT MEANS | 10 |

EP 3 838 150 A1

**Description**

**TECHNICAL FIELD**

The present invention relates to an atrial fibrillation detection system.

**BACKGROUND ART**

Atrial fibrillation is a heart disease involving fine quivering of the atrium of the heart. With this condition, blood can more readily stagnate in the atrium and form a blood clot, and there is a risk that this blood clot could suddenly move to the brain and cause a stroke.

According to epidemiological studies performed by the Japanese Circulation Society, the prevalence of atrial fibrillation was 0.1% for subjects in their 40s, 0.6% for those in their 50s, 0.9% for those in their 60s, and 2.7% for those in their 70s. In addition, according to the Framingham Study, the prevalence of this condition in males aged 65-84 was 3.2% in 1970, but increased over time to 9.1% in 1998.

Because blood clots produced in the atrium due to atrial fibrillation reach a comparatively large size, there is said to be a strong possibility that thick blood vessels in the brain could be blocked and serious aftereffects could remain; however, because atrial fibrillation can be treated, it is possible to maximally reduce the risk that serious illnesses such as stroke will occur if the atrial fibrillation can be discovered at an early stage.

Treatments for atrial fibrillation are broadly divided into drug treatments, in which anti-coagulants, etc., are administered, and catheter treatments (catheter ablation). It is said that about 80% of catheter treatments yield complete recovery from early-stage atrial fibrillation. Although it is early-stage atrial fibrillation that can be completely recovered from through treatment, atrial fibrillation at this stage is referred to as paroxysmal atrial fibrillation, and it is rare for this condition to be discovered in a typical 30-second electrocardiogram (ECG) performed at a hospital or in a diagnostic test.

According to the STROKESTOP Study, most people with this condition are found by intermittently continuing to take ECGs over 15 days, and 60% of people with the condition are found in five days. Specifically, it can be said that measuring ECGs over several days is effective in detecting paroxysmal atrial fibrillation. For example, in diagnostic tests, paroxysmal atrial fibrillation can be detected if ECGs are measured successively over several days, and the occurrence of serious illness such as stroke can be prevented.

However, preparation of machinery for examining several hundred people at the same time in the manner of diagnostic tests, and analysis of ECGs measured over a long period of time, would require great cost and is not practically feasible.

The applicant proposes an atrial fibrillation detection system that detects atrial fibrillation from only the pulse intervals of a heart, such as is shown in Patent Document 1.

Because this atrial fibrillation detection system (referred to as a conventional system below) detects atrial fibrillation from only pulse intervals, the amount of data is lower than that in ECGs even when the pulse intervals are measured over the course of several days, and the cost is lower than that involved in analyzing ECGs. In addition, a device that measures only pulse intervals can be configured to be less expensive than a Holter ECG, and therefore a combination of a device that measures the pulse intervals of a heart and a system that detects atrial fibrillation from only pulse intervals is suitable for diagnostic tests.

However, this conventional system uses a difference between adjacent pulse intervals to detect atrial fibrillation. Therefore, when an extrasystole other than atrial fibrillation is to be excluded, the pulse interval related to the extrasystole and the pulse intervals adjacent thereto must be excluded at the same time, and in cases where this exclusion operation is required, there is a concern that a false-positive assessment result will be detected due to the decrease in the number of pulse intervals.

[Prior-art Documents]

[Patent Document 1] Japanese Patent No. 6150825

**DISCLOSURE OF THE INVENTION**

**PROBLEMS TO BE SOLVED BY THE INVENTION**

The present invention was contrived in view of the aforementioned problems in the conventional system, it being an object of the present invention to provide an atrial fibrillation detection system in which: the extent of irregularity in measured pulse intervals is calculated; even in cases where an extrasystole has occurred, detection of a false positive is reduced even when the extrasystole is not excluded; and highly reliable assessment results are obtained.

**MEANS FOR SOLVING THE PROBLEM**

The main points of the present invention are described below with reference to the accompanying drawings.

The invention according to a first aspect relates to an atrial fibrillation detection system that detects whether atrial fibrillation is occurring in a subject, the atrial fibrillation detection system being characterized in comprising: pulse interval measurement means 4 that measures the pulse intervals of a heart; pulse interval conversion means 8 that performs conversion, using a prescribed function, so that the extent of variation in the pulse intervals R obtained by the pulse interval measurement means 4 is substantially fixed; entropy computation means 9 that calculates entropy S from pulse interval images r obtained through conversion by the pulse interval conversion means 8; and comparative assessment means 10 that compares the entropy S calculated by the entropy computation means 9 and a prescribed threshold value, and that, in cases where the entropy S is greater than the threshold value, assesses that atrial fibrillation is occurring.

The invention according to a second aspect relates to the atrial fibrillation detection system according to the first aspect, characterized in that the pulse interval conversion means 8 is configured so as to convert the pulse intervals R to the pulse interval images r using formula (1).

[Mathematical formula 1]

$$r = f(R) = \alpha \log R + \beta R + \gamma \qquad \cdots (1)$$

In the formula, $\alpha$, $\beta$, and $\gamma$ are constants.

The invention according to a third aspect relates to the atrial fibrillation detection system according to either one of the first and second aspects, characterized in that the entropy computation means 9 is configured so as to calculate the entropy S as described below:

A pulse interval image space of the pulse interval conversion means 8 is divided into a prescribed number M of segments, and the entropy S is calculated from the N pulse interval images r obtained by the pulse interval conversion means 8 according to formula (2).

[Mathematical formula 2]

$$S = \log N! - \sum_{m=1}^{M} \log n_m! \qquad \cdots (2)$$

In the formula, $n_m$ refers to the number of pulse interval images r included in an $m^{th}$ segment.

The invention according to a fourth aspect relates to the atrial fibrillation detection system according to either one of the first and second aspects, characterized in that the entropy computation means 9 is configured so as to calculate the entropy S as described below:

A pulse interval image space of the pulse interval conversion means 8 is divided into a prescribed number M of segments, and the entropy S is calculated from the N pulse interval images r obtained by the pulse interval conversion means 8 according to formula (3).

[Mathematical formula 3]

$$S = - \sum_{m=1}^{M} \frac{n_m}{N} \log \frac{n_m}{N} \qquad \cdots (3)$$

In the formula, $n_m$ refers to the number of pulse interval images r included in an $m^{th}$ segment.

The invention according to a fifth aspect relates to the atrial fibrillation detection system according to either one of the first and second aspects, characterized in that the entropy computation means 9 is configured so as to calculate the entropy S as described below:

Prescribed distributions g are kept centered on the values of each of the N pulse interval images r obtained by the pulse interval conversion means 8, a distribution G obtained from the sum of the N distributions g is normalized using formula (4), a probability density distribution p is calculated using formula (5), and the entropy S is calculated using formula (6).

[Mathematical formula 4]

$$G(r) = \sum_{n=1}^{N} g(r, r_n) \qquad \cdots (4)$$

[Mathematical formula 5]

$$p(r) = \frac{G(r)}{\int_{r_1}^{r_2} G(r)dr} \qquad \cdots (5)$$

[Mathematical formula 6]

$$S = -\int_{r1}^{r2} p(r) \log p(r) \, dr \qquad \cdots (6)$$

In the formulas, r is a variable in the pulse interval image space of the pulse interval conversion means 8, and $r_1$ and $r_2$ are the lower end and upper end of a prescribed integral segment.

The invention according to a sixth aspect relates to the atrial fibrillation detection system according to the fifth aspect, characterized in that the distribution g is represented by formula (7) .

[Mathematical formula 7]

$$g(r, r_n) = c \exp\left\{-\frac{(r - r_n)^2}{2\sigma^2}\right\} \qquad \cdots (7)$$

In the formula, r is a variable in the pulse interval image space of the pulse interval conversion means 8, and $r_n$ is a pulse interval image r converted by the pulse interval conversion means 8. In addition, c is an arbitrary constant other than 0, and $\sigma$ is a standard deviation representing spreading of the distribution.

The invention according to a seventh aspect relates to the atrial fibrillation detection system according to any one of the first to sixth aspects, characterized in being configured so that: the pulse interval measurement means 4 is provided to a pulse interval measurement sensor 2, and the pulse interval conversion means 8, the entropy computation means 9, and the comparative assessment means 10 are provided to an analyzer 3.

The invention according to an eighth aspect relates to the atrial fibrillation detection system according to the seventh aspect, characterized in being configured so that: the pulse interval measurement sensor 2 has pulse interval transmission means 5 that transmits the pulse intervals R to the analyzer 3, or a pulse interval preservation means 11 that preserves the pulse intervals R; and the pulse intervals R are inputted to the analyzer 3 via the pulse interval transmission means 5 or the pulse interval preservation means 11 provided to the pulse interval measurement sensor 2, whereby atrial fibrillation is detected.

The invention according to a ninth aspect relates to the atrial fibrillation detection system according to any one of the first to eighth aspects, characterized in comprising extrasystole exclusion means 7 that excludes pulse intervals derived from extrasystoles from among the pulse intervals R measured by the pulse interval measurement means 4, and being configured so that the entropy S is calculated by the entropy computation means 9 from the pulse interval images r obtained through conversion by the pulse interval conversion means 8 so that the extent of variation in the pulse intervals R, from which pulse intervals derived from extrasystoles have been excluded by the extrasystole exclusion means 7, is substantially fixed.

The invention according to a tenth aspect relates to the atrial fibrillation detection system according to the ninth aspect, characterized in that the extrasystole exclusion means 7 is configured so as to exclude pulse intervals derived from extrasystoles as described below on the basis of the magnitude of the difference between the pulse intervals R measured by the pulse interval measurement means 4 and the average value of the pulse intervals R.

The invention according to an eleventh aspect relates to the atrial fibrillation detection system according to the tenth aspect, characterized in that the extrasystole exclusion means 7 is configured so as to exclude pulse intervals derived from extrasystoles as described below:

Pulse intervals $R_n$ and $R_{n+1}$ that satisfy formulas (9) through (11) are excluded, where $R_n$ is a time series of pulse intervals R measured by the pulse interval measurement means 4, formula (8) indicates a normalized pulse interval NDRn, and $R_n$ is the average value calculated using a prescribed method.

[Mathematical formula 8]

$$NDR_n = \frac{2(R_n - R_{n+1})}{R_n + R_{n+1}} \qquad \cdots (8)$$

In the formula, n represents a time series, and refers to the past relative to n+1.

[Mathematical formula 9]

$$NDR_{n-1} > A, \qquad NDR_n < -B \qquad \cdots (9)$$

[Mathematical formula 10]

$$\frac{R_n}{\bar{R}_n} < C, \qquad \frac{R_{n+1}}{\bar{R}_{n+1}} > D, \qquad \cdots (10)$$

[Mathematical formula 11]

$$E < \frac{R_n + R_{n+1}}{\bar{R}_n + \bar{R}_{n+1}} < F \qquad \cdots (11)$$

In the formulas, each of the threshold values A, B, C, D, E, and F is a value greater than 0, and E < F.

## EFFECT OF THE INVENTION

Due to being configured as described above, the present invention is a useful atrial fibrillation detection system in which: the extent of irregularity in measured pulse intervals is calculated, and even in cases where an extrasystole has occurred, detection of a false positive is reduced even when the extrasystole is not excluded; and highly reliable assessment results are obtained.

## BRIEF DESCRIPTION OF THE DRAWINGS

FIG. 1 is a configuration overview diagram showing example 1;
FIG. 2 is a configuration overview diagram showing example 2;
FIG. 3 is a configuration overview diagram showing example 3;
FIG. 4 is a configuration overview diagram showing another aspect of example 3;
FIG. 5 is a configuration overview diagram showing another aspect of example 3;
FIG. 6 is a graph showing a distribution of the difference between pulse intervals relative to an average of adjacent pulse intervals in patients with atrial fibrillation;
FIG. 7 is a graph showing a distribution of normalized pulse intervals relative to an average of adjacent pulse intervals R in patients with atrial fibrillation;
FIG. 8 is a histogram of normalized pulse intervals in healthy subjects;
FIG. 9 is a histogram of pulse interval images for 21 beats in healthy subjects and in patients with atrial fibrillation;
FIG. 10 is a graph showing the probability density of the pulse interval images for 21 beats in healthy subjects and in patients with atrial fibrillation;

FIG. 11 is a graph showing a time series of pulse intervals in which there is an extrasystole, and an average value of the time series;

FIG. 12 is a graph showing that it is possible to exclude the extrasystole from the pulse intervals in which there is an extrasystole;

FIG. 13 is a table showing entropy obtained from formula (14) for 21 beats in healthy subjects and in patients with atrial fibrillation;

FIG. 14 is a table showing entropy obtained from formula (15) for 21 beats in healthy subjects and in patients with atrial fibrillation;

FIG. 15 is a table showing entropy obtained from formula (18) for 21 beats in healthy subjects and in patients with atrial fibrillation; and

FIG. 16 is a table showing, for subjects having extrasystoles and for patients with atrial fibrillation, assessment results obtained using the atrial fibrillation detection system disclosed in Patent Document 1, the entropy obtained from formula (18), and the entropy in a case where an extrasystole exclusion means is used.

**BEST MODE FOR CARRYING OUT THE INVENTION**

Preferred embodiments of the present invention are briefly described below with reference to the diagrams while indicating the effects of the present invention.

A prescribed number (e.g., at least about 20 beats) of pulse intervals R measured by pulse interval measurement means 4 are converted by pulse interval conversion means 8 using a prescribed function whereby the extent of variation in the pulse intervals R is substantially fixed, and pulse interval images r are formed. Entropy S indicating the extent of irregularity in the pulse interval images r is calculated by entropy computation means 9. The entropy S is compared with a prescribed threshold value by comparative assessment means 10, and in cases where the entropy S is greater than the threshold value, it is assessed that atrial fibrillation is occurring.

Because there is a certain regularity in extrasystoles other than atrial fibrillation, increases in the entropy S due thereto is not caused by atrial fibrillation. Therefore, the present invention achieves an atrial fibrillation detection system with which it is possible to reduce false positives in detection of atrial fibrillation even when pulse intervals R related to extrasystoles other than atrial fibrillation are not excluded, and to obtain highly reliable assessment results.

Moreover, because it is possible, in the present invention, to detect whether atrial fibrillation is occurring in pulse intervals R for several tens of beats as described above, it is possible to detect symptoms of atrial fibrillation in a short period of time, and thus it is possible to mitigate the burden on a subject and to detect paroxysmal atrial fibrillation occurring in a short period of time.

Furthermore, because the present invention can calculate entropy S from the pulse intervals R, the present invention achieves a useful atrial fibrillation detection system in which it is not necessary to use a Holter ECG, a 12-lead resting ECG, or another dedicated device in which electrode positions are strictly designated, and in which it is possible, for example, to calculate entropy S from the pulse intervals R obtained using a small and convenient household measurement instrument such as a cardiometer affixed to the chest or a wristband-type sphygmograph, and to detect whether atrial fibrillation is occurring.

[Example 1]

A specific example 1 of the present invention is described below with reference to the diagrams.

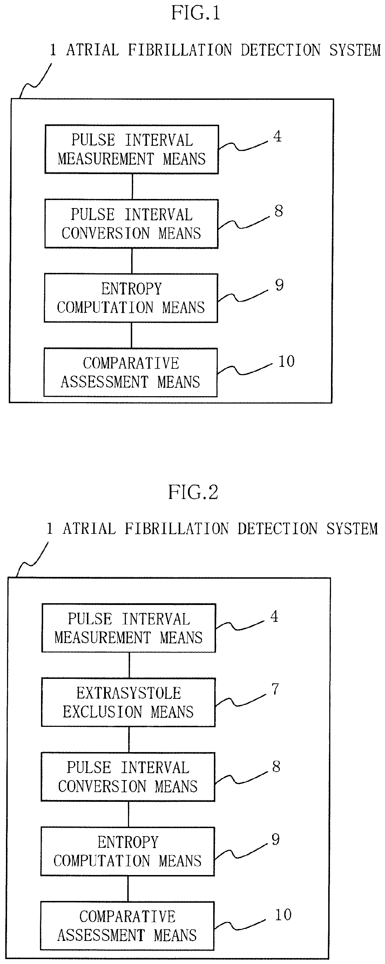

The present example is an atrial fibrillation detection system 1 that detects whether atrial fibrillation is occurring in a subject, the atrial fibrillation detection system 1 comprising, as shown in FIG. 1: pulse interval measurement means 4 that measures the pulse intervals of a heart; pulse interval conversion means 8 that performs conversion, using a prescribed function, so that the extent of variation in the pulse intervals R obtained by the pulse interval measurement means 4 is substantially fixed; entropy computation means 9 that calculates entropy S from pulse interval images r obtained through conversion by the pulse interval conversion means 8; and comparative assessment means 10 that compares the entropy S calculated by the entropy computation means 9 and a prescribed threshold value, and that, in cases where the entropy S is greater than the threshold value, assesses that atrial fibrillation is occurring.

The configuration elements according to the present example are described in detail below.

The pulse interval measurement means 4 is configured so as to, for example, measure the intervals between adjacent R waves, or measure a pulse interval R from the intervals between adjacent S waves, from an ECG performed on the basis of a variation in voltage obtained from electrodes using a microcomputer, etc. The pulse interval measurement means 4 may also be configured so as to measure pulse waves from reflected light of infrared rays, and measure the pulse intervals R from, *inter alia,* the peak intervals of the measured pulse waves. Alternatively, the pulse interval measurement means 4 may be configured so as to capture a heartbeat or pulse sound and electrically process one or both of the heartbeat and the pulse sound to measure the pulse interval R.

The pulse interval conversion means 8 is configured so as to perform conversion, using a prescribed function, to a distribution of pulse interval images r such that the extent of variation in the pulse intervals R of the heart of a subject (i.e., the distribution of the pulse intervals R) is substantially fixed (a substantially symmetrical distribution), the pulse intervals R having been obtained through measurement by the pulse interval measurement means 4 described above.

Specifically, formula (12), for example, can be employed as the prescribed function. Formula (12) is the same as formula (1).

[Mathematical formula 12]

$$r = f(R) = \alpha \log R + \beta R + \gamma \qquad \cdots (12)$$

In the formula, $\alpha$ is a coefficient other than 0, $\beta$ is a coefficient that may be 0, and $\gamma$ is an arbitrary constant.

The pulse interval conversion means 8 in the present example is more specifically described below.

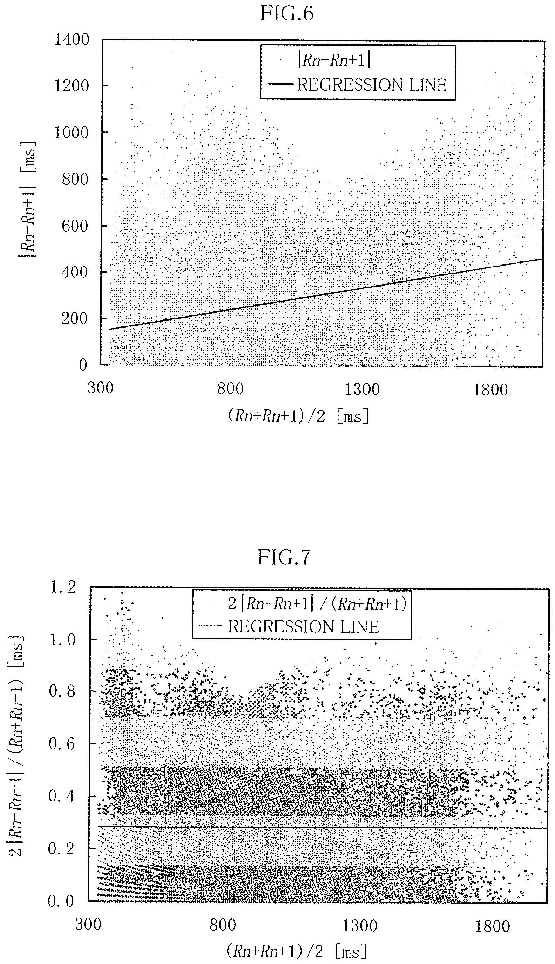

FIG. 6 shows a relationship between the magnitude of the average of adjacent pulse intervals R in 39,061 items of pulse interval value data for patients with atrial fibrillation and the magnitude of the difference between the pulse intervals R. In FIG. 6, the magnitude $R_n$ of the average of adjacent pulse intervals R is plotted on the horizontal axis such that $R_n = (R_n + R_{n+1})/2$, and the magnitude $|\Delta R_n|$ of the difference between the pulse intervals R is plotted on the vertical axis such that $|\Delta R_n| = |R_n - R_{n+1}|$ ("||" is the notation for absolute value).

The horizontal axis in FIG. 6 can be regarded as the magnitude of the pulse intervals R and the vertical axis in FIG. 6 can be regarded as the extent of variation in the pulse intervals R, and therefore, because the extent of variation in the pulse intervals R tends to increase together with the pulse intervals R as shown by the regression line in the drawing, it is understood that the extent of irregularity depends on the magnitude of the pulse intervals R, and that the breadth of the distribution of the pulse intervals R increases as the pulse intervals R increase.

Typically, entropy S increases commensurately with increases in distribution; therefore, it can be said that: when entropy S is directly calculated from the distribution of the pulse intervals R, the magnitude of the entropy S depends on the magnitude of the pulse intervals R; and if the entropy S is used when assessing whether atrial fibrillation is occurring, it is undesirable for the sensitivity to depend on the magnitude of the pulse intervals R.

To calculate the entropy S without being affected by the magnitude of the pulse intervals R, it is preferable to adopt a configuration in which the pulse intervals R are converted using a prescribed function and in which the extent of variation in pulse interval images r obtained through this conversion does not depend on the pulse intervals R. The pulse interval images r obtained in this manner are fixed so as not to depend on the magnitude of the pulse intervals R, and when the entropy S is calculated from the pulse interval images r, entropy S that does not depend on the magnitude of the pulse intervals R is obtained.

FIG. 7 shows a relationship between the magnitude of the average of two pulse intervals R in the same items of pulse interval value data as are used in the plotting of FIG. 6 and the absolute value of a normalized pulse interval, which is a value obtained by dividing the magnitude of the difference between the pulse intervals R by the average value of the pulse intervals R. In FIG. 7, the magnitude $R_n$ of the average of adjacent pulse intervals R is plotted on the horizontal axis, and the absolute value $|NDR_n|$ of the normalized pulse interval is plotted on the vertical axis such that $|NDR_n| \equiv 2|R_n - R_{n+1}|/(R_n + R_{n+1})$ ("||" is the notation for absolute value).

Because the extent of variation in the normalized pulse intervals substantially does not depend on the pulse intervals R as shown by the regression line in FIG. 7, the normalized pulse intervals on the vertical axis are expressed as $R_n - R_{n+1} = dR$ and $(R_n + R_{n+1})/2 = R$, as $\Delta R_n \to 0$, and the extent of variation in the pulse interval images r obtained using the function in formula (13) in which dR/R is integrated should be fixed and not depend on the pulse intervals R.

[Mathematical formula 13]

$$r = f(R) = \alpha \log R + \gamma \qquad \cdots (13)$$

$\alpha$ is a coefficient other than 0, and $\gamma$ is an arbitrary constant.

A slight dependency on the pulse intervals can be seen in the regression line shown in FIG. 7. In such instances, the pulse interval difference $\Delta R_n$ in FIG. 6 described above may be added at a given percentage to the normalized pulse intervals $NDR_n$ in FIG. 7 to make a correction.

The value obtained in this manner is $\alpha NDR_n + \beta \Delta R_n$, and therefore: this value can be expressed as $(\alpha/R + \beta)dR$, as $\Delta R_n \to 0$; the function in formula (12) described above is obtained by integrating this value; and the extent of variation in the pulse interval images r obtained by conversion in the function of formula (12) depends even less on the pulse intervals R.

The entropy computation means 9 is configured so as to calculate the extent of irregularity in the pulse interval images r obtained by the pulse interval conversion means 8 described above, and specifically is configured so as to calculate the entropy S from a prescribed number of successive pulse interval images r obtained by the pulse interval conversion means 8.

The entropy computation means 9 of the present example is more specifically described below.

The pulse interval image space of the pulse interval conversion means 8 is divided into M segments, and a histogram of N pulse interval images r is created, where N is a prescribed number of pulse interval images r obtained by the pulse interval conversion means 8. In this histogram, the entropy S can be written according to formula (14), where $n_m$ is the number of pulse interval images r in an $m^{th}$ segment. The right-side portion of formula (14) is the same as formula (2).

[Mathematical formula 14]

$$S = \log\left(N! \Big/ \prod_{m=1}^{M} n_m\right) = \log N! - \sum_{m=1}^{M} \log n_m! \qquad \cdots (1\,4)$$

If formula (14) is divided by N and furthermore transformed using Stirling's formula to obtain formula (15), the entropy S that does not depend on the magnitude of the pulse interval image number N will be obtained. Formula (15) is the same as formula (3).

[Mathematical formula 15]

$$S = -\sum_{m=1}^{M} \frac{n_m}{N} \log \frac{n_m}{N} \qquad \cdots (1\,5)$$

In calculation of the entropy S, the prescribed pulse interval image number N is preferably set to about 20-100, thereby making it possible to detect paroxysmal atrial fibrillation as well. It is preferable for the segments in the pulse interval image space to be equally divided using function f in formula (12) so that the number of segments M from f(300) to f(2000) equals 17.

It is also possible to keep prescribed distributions g centered on the values of each of the N pulse interval images r obtained by the pulse interval conversion means, normalize a distribution G obtained from the sum of the N distributions g using formula (16), calculate a probability density distribution p using formula (17), and calculate the entropy S using formula (18). Formula (16) is the same as formula (4), formula (17) is the same as formula (5), and formula (18) is the same as formula (6).

[Mathematical formula 16]

$$G(r) = \sum_{n=1}^{N} g(r, r_n) \qquad \cdots (1\,6)$$

[Mathematical formula 17]

$$p(r) = \frac{G(r)}{\int_{r_1}^{r_2} G(r) dr} \qquad \cdots (1\,7)$$

[Mathematical formula 18]

$$S = -\int_{r1}^{r2} p(r) \log p(r)\, dr \qquad \cdots (1\,8)$$

In the formulas, $r_1$ and $r_2$ are the lower end and upper end of an integral segment for which $r_1 < r_2$ holds. The lower end and upper end may be set from $r_1 = f(300)$ to $r_2 = f(2000)$ using the function f in formula (12) described above, or may be set from $r_1 = 0$ to $r_2 = +\infty$.

The entropy S obtained in this manner is a universal entropy S because it is not necessary to divide the pulse interval image space into M segments. It is suitable to use a normal distribution as the distribution $g(r, r_n)$ in formula (16); specifically, formula (19), for example, can be used.

[Mathematical formula 19]

$$g(r, r_n) = c \exp\left\{-\frac{(r - r_n)^2}{2\sigma^2}\right\} \qquad \cdots (1\,9)$$

In this formula, c is an arbitrary constant other than 0, and $\sigma$ is a standard deviation representing spreading of the distribution. The arbitrary constant c is eliminated in formula (17), and therefore 1 can be employed for this arbitrary constant. The standard deviation of the normalized pulse interval NDR of a healthy subject may be employed as the standard deviation $\sigma$.

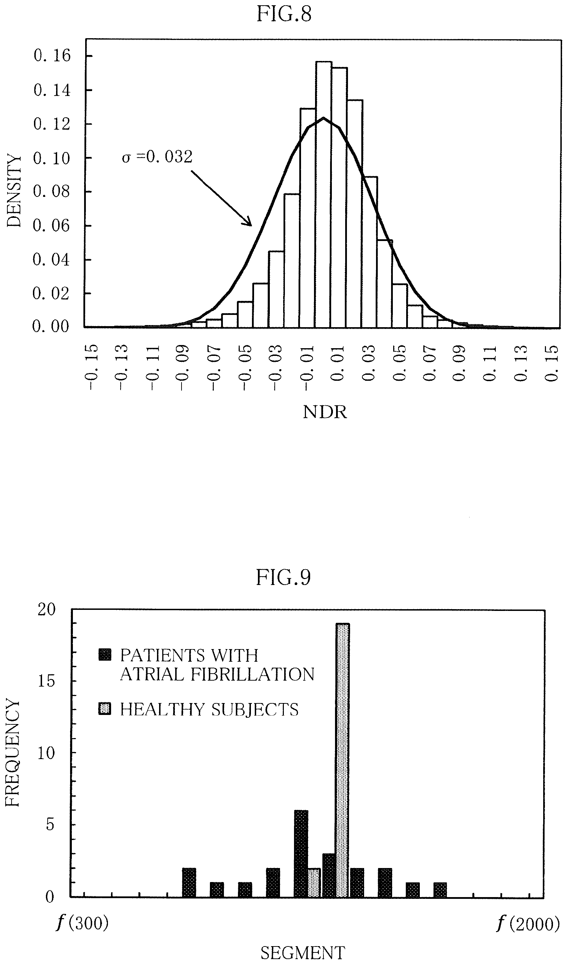

FIG. 8 shows the distribution of normalized pulse intervals NDR obtained from 74,257 healthy subjects. Because the standard deviation in this distribution is 0.032, a value from 0.02 to about 0.06 is suitable as the standard deviation $\sigma$ in formula (19).

The comparative assessment means 10 is configured so as to compare the entropy S calculated by the entropy computation means 9 and a prescribed threshold value, and, when the entropy S is greater than the threshold value, to assess that atrial fibrillation is occurring.

It is indicated next that using the atrial fibrillation detection system 1 of the present example configured as described above makes it possible to detect whether a subject has atrial fibrillation from pulse intervals R for 21 beats in each of 20 examples of patients with actual atrial fibrillation and 20 examples of healthy subjects.

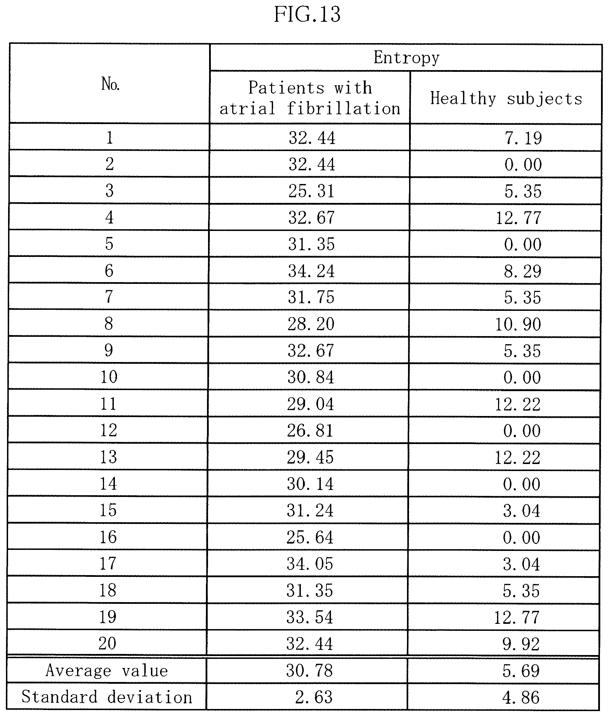

First, the pulse intervals R are converted using formula (12). In this instance, the coefficients in formula (12) are set such that $\alpha = 1$, $\beta = 0$, and $\gamma = 0$. Segments of the pulse interval image space are divided into 17 parts (number of segments M = 17) from f(300) to f(2000) using the function f in formula (12). The number $n_m$ of pulse interval images in the $m^{th}$ segment is calculated. A histogram of pulse interval images r for 21 beats in patients with atrial fibrillation and in healthy subjects is shown in FIG. 9. It is anticipated that the entropy S in a patient with atrial fibrillation will be high. According to formula (14), the entropy S in the 20 examples of patients with atrial fibrillation and the 20 examples of healthy subjects is as shown in FIG. 13. From the average values and standard deviations in patients with atrial fibrillation and in healthy subjects, it can be assessed with 99.96% certainty whether atrial fibrillation is occurring if the threshold value is set to 21.97.

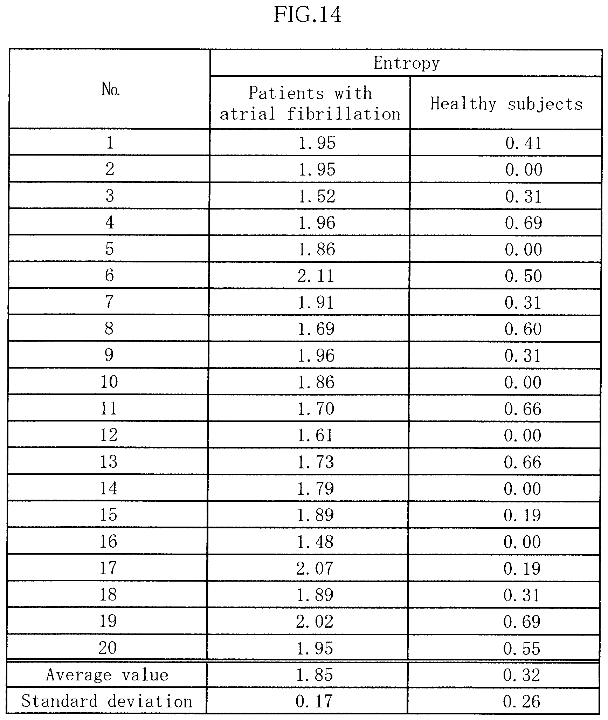

The entropy S calculated using formula (15) is as shown in FIG. 14. From the average values and standard deviations in patients with atrial fibrillation and in healthy subjects in FIG. 14, it can be assessed with 99.97% certainty whether atrial fibrillation is occurring if the threshold value is set to 1.24.

In the calculation of the entropy S using formula (14) or formula (15), the resulting values differ depending on how a segment from a particular start and end point of the pulse interval image space is divided. A case is illustrated below in which the entropy S is calculated in accordance with formula (18), which does not depend on the method for dividing segments.

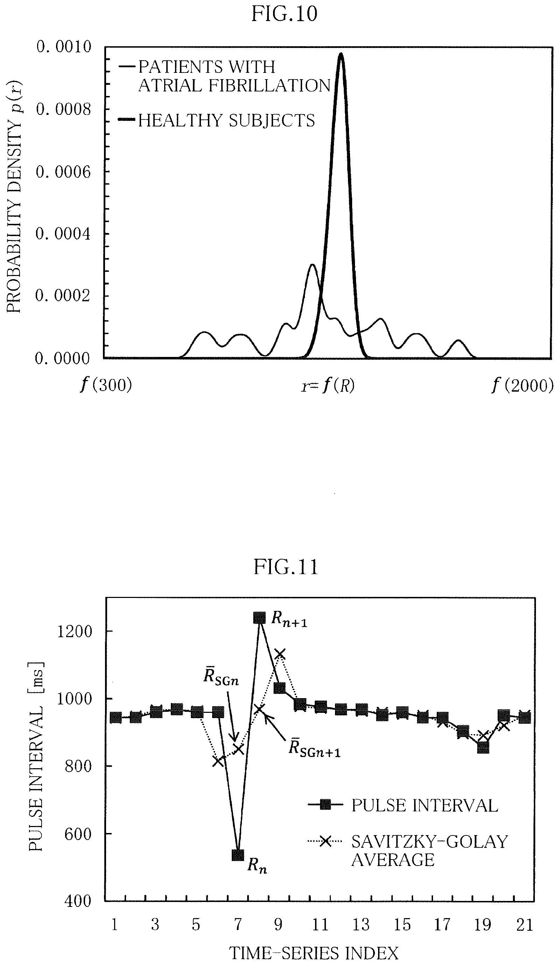

In the same manner as in the cases in which formulas (14) and (15) were used as described above, the pulse intervals R are converted using formula (12) assuming $\alpha = 1$, $\beta = 0$, and $\gamma = 0$. The standard deviation in formula (19) is next set so that $\sigma = 0.032$, and the probability density distribution p is derived from formulas (16) and (17). The probability density distribution p obtained, using formula (17), from the pulse interval images r for 21 beats in patients with atrial fibrillation and in healthy subjects is shown in FIG. 10. It is understood that because the result in FIG. 10 is smoother than the histogram in FIG. 9, it is possible to perform integration irrespective of segments.

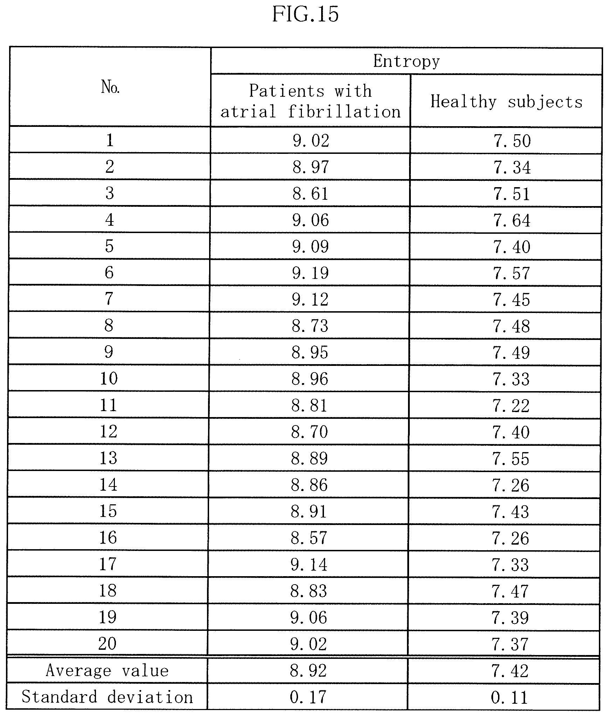

The entropy S calculated using formula (18) is as shown in FIG. 15. From the average values and standard deviations in patients with atrial fibrillation and in healthy subjects in FIG. 15, it can be assessed with 100% certainty whether atrial fibrillation is occurring if the threshold value is set to 8.01.

Thus, the present example achieves a useful atrial fibrillation detection system in which: the extent of irregularity in measured pulse intervals R is calculated, and thus even in cases where an extrasystole has occurred, detection of a false positive can be reduced even when the extrasystole is not excluded; and highly reliable assessment results can be obtained.

[Example 2]

A specific example 2 of the present invention is described below with reference to the diagrams.

The present example shows a case where the atrial fibrillation detection system in example 1 is configured so as to furthermore comprise extrasystole exclusion means 7 that excludes pulse intervals R derived from extrasystoles other than atrial fibrillation, and to have a lower incidence of erroneous detection (false positives).

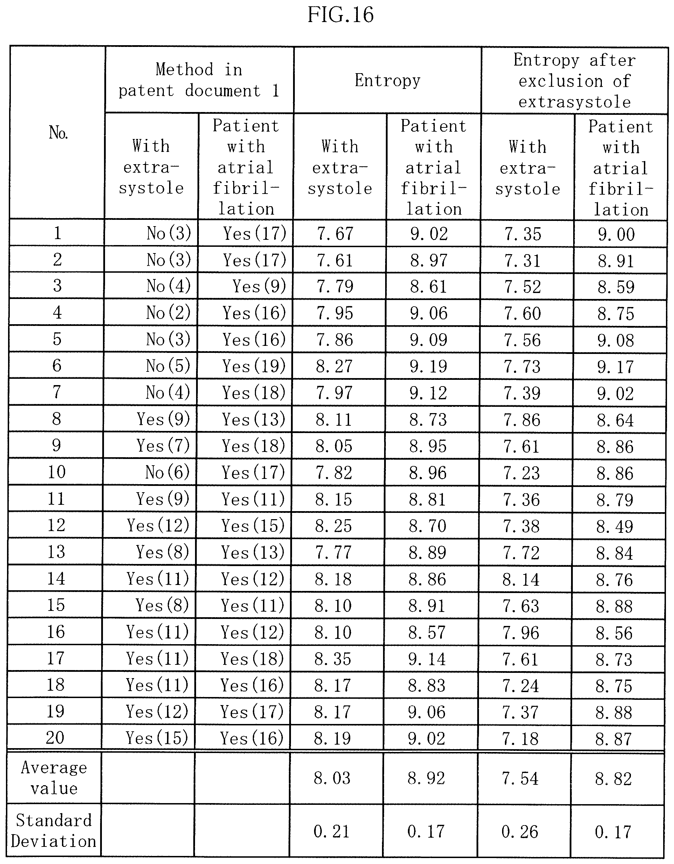

Specifically, in the atrial fibrillation detection system of Patent Document 1 (Japanese Patent No. 6150825), which is indicated as prior art, a normalized pulse interval NDR obtained through dividing the difference between adjacent pulse intervals R by the average value thereof is introduced, results in which the absolute value of the normalized pulse interval is greater than 0.1 are designated as abnormal normalized pulse intervals, and, when there are seven or more abnormal normalized pulse intervals among 20 successive normalized pulse intervals, a patient with atrial fibrillation can be distinguished, with the highest sensitivity and specificity, from a healthy subject. In this method, if there are extrasystoles other than atrial fibrillation, the absolute value of normalized pulse intervals calculated from pulse intervals related to these extrasystoles are greater than those calculated from normal sinus rhythms, and therefore there are cases where atrial fibrillation is erroneously detected. As pertains to 20 examples of subjects with extrasystoles other than atrial fibrillation, with supraventricular premature contraction and ventricular premature contraction being included among these extrasystoles, and 20 examples of patients with atrial fibrillation, results obtained by assessing whether atrial fibrillation was occurring using the method described above are shown in the left-side columns, under "Method in patent document 1," in FIG. 16. Subjects for whom it was assessed that atrial fibrillation was occurring were displayed as "Yes," and subjects for whom this was not the case were displayed as "No." The numeric values in parentheses following these terms are the number of abnormal normalized pulse intervals from among 20 normalized pulse intervals. These results made it possible to distinguish all of the patients with atrial fibrillation, but it is understood that atrial fibrillation was erroneously detected in 12 examples among the 20 examples of subjects having other extrasystoles.

It is indicated next that when the entropy S is calculated in accordance with formula (18) in example 1, the result is less likely to be affected by extrasystoles other than atrial fibrillation. For the purposes of formula (18), the standard deviation in formula (19) is set so that $\sigma = 0.032$, the probability density distribution p is derived from formulas (16) and (17), and the pulse intervals R are converted using formula (12) assuming $\alpha = 1$, $\beta = 0$, and $\gamma = 0$. As a result, the average values of the entropy S for subjects including extrasystoles and patients with atrial fibrillation were 8.03 and 8.92, respectively, and the standard deviations for these groups were 0.21 and 0.17, respectively, as shown in the center columns in FIG. 16. Therefore, atrial fibrillation can be distinguished with 99.0% certainty if the threshold value is set to 8.52; specifically, it is indicated that the assessment is not likely to be affected by extrasystoles other than atrial fibrillation.

However, even if the entropy S is used, it is thought that assessment precision will worsen in cases where a significantly large number of extrasystoles other than atrial fibrillation are included in the pulse intervals R. Examples of extrasystoles include supraventricular premature contraction, ventricular premature contraction, sinus arrest, and atrioventricular block, in addition to atrial fibrillation, and because these conditions also entail large entropy S, these conditions cause the specificity of atrial fibrillation detection to worsen.

In the present example, in order to prevent worsening of assessment precision due to such inclusion of many extrasystoles other than atrial fibrillation, the atrial fibrillation detection system 1 in example 1 above is configured so as to furthermore comprise the extrasystole exclusion means 7 that excludes pulse intervals R derived from extrasystoles other than atrial fibrillation, and to have a lower incidence of erroneous detection (false positives).

Specifically, as shown in FIG. 2, the present example comprises: pulse interval measurement means 4 that measures the pulse intervals of a heart; extrasystole exclusion means 7 that excludes pulse intervals derived from extrasystoles from among the pulse intervals R obtained by the pulse interval measurement means 4; pulse interval conversion means 8 that performs conversion, using a prescribed function, so that the extent of variation in the pulse intervals R from which the pulse intervals derived from extrasystoles have been excluded by the extrasystole exclusion means 7 is substantially fixed; entropy computation means 9 that calculates entropy S from pulse interval images r obtained through conversion by the pulse interval conversion means 8; and comparative assessment means 10 that compares the entropy S calculated by the entropy computation means 9 and a prescribed threshold value, and that, in cases where the entropy S is greater than the threshold value, assesses that atrial fibrillation is occurring. Because the pulse interval measurement means 4, the pulse interval conversion means 8, the entropy computation means 9, and the comparative assessment means 10 in the present example are the same as those in example 1, only the extrasystole exclusion means 7 is described here.

The extrasystole exclusion means 7 of the present example is configured so as to exclude ventricular and atrial extrasystoles.

Specifically, the extrasystole exclusion means 7 is configured so as to calculate the average value of a prescribed number of pulse intervals R using a prescribed method, and to exclude the pulse intervals on the basis of the magnitude of the difference between the pulse intervals R and the average value of the pulse intervals R.

In the extrasystole exclusion means 7, various methods can be employed in order to calculate the average value of the pulse intervals R. For example, an average value corresponding to the pulse interval $R_n$ may be employed, or a moving average of a total of approximately K pulse intervals R that include the pulse interval $R_n$ indicated in formula (20) may be employed.

[Mathematical formula 20]

$$\bar{R}_{MVn} = \frac{1}{K} \sum_{k=n-\frac{K-1}{2}}^{n+\frac{K-1}{2}} R_{n+k} \qquad \cdots (20)$$

The Savitzky-Golay method, which has an effect for excluding high-frequency noise, can also be used. For example, the average of pulse intervals R where K = 5 is shown in formula (21).

[Mathematical formula 21]

$$\bar{R}_{SGn} = \frac{-3R_{n-2} + 12R_{n-1} + 17R_n + 12R_{n+1} - 3R_{n+2}}{35} \qquad \cdots (21)$$

FIG. 11 shows one example of an extrasystole. The square marks linked by solid lines in FIG. 11 represent the pulse intervals R, and the × marks linked by dotted lines represent the average value obtained from formula (21) according to the Savitzky-Golay method. When an extrasystole occurs, two successive pulse intervals R are respectively shorter and longer than a normal sinus rhythm. Specifically, the heart undergoes a premature contraction at the pulse interval $R_n$ in FIG. 11, and a long pulse interval $R_{n+1}$ occurs due to a compensatory pause.

The pulse interval $R_n$ at the premature contraction is about 10% shorter than the pulse intervals in a normal sinus rhythm, and the long pulse interval $R_{n+1}$ at the compensatory pause is about 10% longer than a normal sinus rhythm. Therefore, the following normalized pulse intervals $NDR_{n-1}$ and $NDR_n$ in which the pulse intervals $R_n$ and $R_{n+1}$ of the extrasystole are included satisfy formula (22).

[Mathematical formula 22]

$$NDR_{n-1} = \frac{2(R_{n-1} - R_n)}{R_{n-1} + R_n} > A, \quad NDR_n = \frac{2(R_n - R_{n+1})}{R_n + R_{n+1}} < -B \quad \cdots (22)$$

A and B are values greater than 0. In consideration of the characteristics of the extrasystole, a value from 0.05 to about 0.2 is suitable for both A and B.

The average values $R_{SGn}$ and $R_{SGn+1}$ in FIG. 11 are obtained from formula (21) in the Savitzky-Golay method. When there is an extrasystole, the pulse interval $R_n$ at the premature contraction, the pulse interval $R_{n+1}$ at the compensatory pause, and the average values $R_{SGn}$ and $R_{SGn+1}$ have the relationships $R_n < R_{SGn}$ and $R_{SGn+1} < R_{n+1}$, as shall be apparent from FIG. 11. Therefore, if there is an extrasystole, formula (23) is satisfied.

[Mathematical formula 23]

$$\frac{R_n}{\bar{R}_{SGn}} < C, \qquad \frac{R_{n+1}}{\bar{R}_{SGn+1}} > D, \qquad \cdots (23)$$

The threshold values C and D are values greater than 0. In consideration of the fact that the pulse interval $R_n$ at the premature contraction is about 10% shorter than the pulse intervals of a normal sinus rhythm, a value of 0.5-1.0 inclusive is suitable for C. Moreover, in consideration of the fact that the value obtained by subtracting the pulse interval R of a normal sinus rhythm from the difference $R_{n+1} - R_n$ between the pulse intervals at the compensatory pause and at the premature contraction is less than the value obtained by subtracting the pulse interval $R_n$ at the premature contraction from the normal sinus rhythm, a value of 1.0-1.2 inclusive is suitable for D.

Furthermore, because $R_n + R_{n+1}$, which is obtained by adding the pulse intervals at the premature contraction and at the compensatory pause, is substantially equal to double the pulse interval in a normal sinus rhythm, formula (24) is satisfied.

[Mathematical formula 24]

$$E < \frac{R_n + R_{n+1}}{\bar{R}_{SGn} + \bar{R}_{SGn+1}} < F \qquad \cdots (24)$$

In the formula, the threshold values E and F are values satisfying the expression E < F. A value of 0.8-1.0 inclusive is suitable for E, and a value of 1.0-1.2 is suitable for F.

If pulse intervals $R_n$ and $R_{n+1}$ that simultaneously satisfy formulas (22), (23), and (24) in this manner are excluded, it is possible to exclude ventricular and atrial extrasystoles and to improve the sensitivity and specificity of detecting atrial fibrillation.

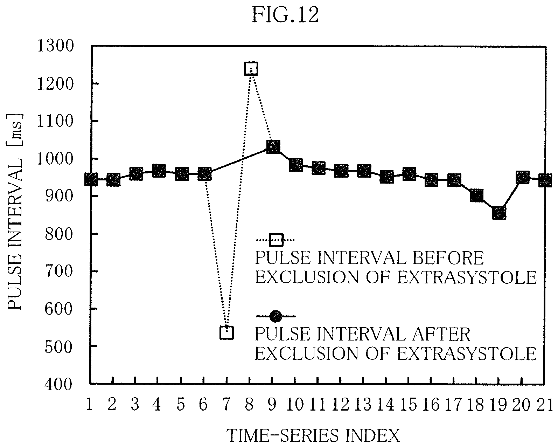

FIG. 12 shows a situation in which ventricular and atrial extrasystoles are excluded from the pulse intervals R that actually include extrasystoles shown in FIG. 11 through the method described above. The empty white squares linked by dotted lines represent a time series of the pulse intervals R before exclusion of the extrasystoles, and the black circles linked by solid lines represent a time series of the pulse intervals R after this exclusion. It is understood from FIG. 12 that the excluded pulse intervals R have premature contractions and compensatory pauses. The threshold values in each of formulas (22) through (24) are set such that A = B = 0.1, C = 0.9, D = 1.05, E = 0.95, and F = 1.1.

The results of investigating the effect of excluding extrasystoles using the pulse intervals R for 21 beats in each of 20 examples of subjects with actual extrasystoles and 20 examples of patients with atrial fibrillation are shown in FIG. 16. The values described above were used for the threshold values A to F in formulas (22) through (24). In the calculation of the entropy S, in order to follow formula (18) shown in example 1, the standard deviation in formula (19) was set so that $\sigma = 0.032$, and the probability density distribution p was derived from formulas (16) and (17). The pulse intervals R were converted using formula (12) assuming $\alpha = 1$, $\beta = 0$, and $\gamma = 0$.

In the results from before exclusion of the extrasystoles is applied (i.e., the results in the center columns in FIG. 16), the average values of the entropy S for subjects including extrasystoles and patients with atrial fibrillation were 8.03 and 8.92, respectively, and the standard deviations were 0.21 and 0.17, respectively, as described above. Therefore, atrial fibrillation can be distinguished with 99.0% certainty if the threshold value is set to 8.52.

Moreover, in the results from after exclusion of the extrasystoles is applied (i.e., the results in the right-side columns in FIG. 16), the average values of the entropy S for subjects including extrasystoles and patients with atrial fibrillation were 7.54 and 8.82, respectively, and the standard deviations were 0.26 and 0.17, respectively. Therefore, atrial fibrillation can be distinguished with a certainty of 99.9% if the threshold value is set to 8.13, and it is understood that there is achieved an atrial fibrillation detection system 1 in which there is a lower incidence of erroneous detection than in cases where exclusion of the extrasystoles is not applied.

[Example 3]

A specific example 3 of the present invention is described below with reference to the diagrams.

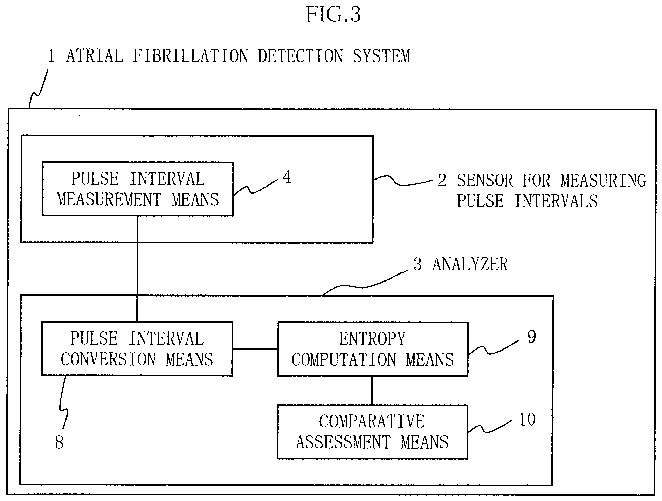

The present example has a different configuration from example 1. Whereas the atrial fibrillation detection system 1 in example 1 has a system configuration (device configuration) in which the pulse interval measurement means 4, the pulse interval conversion means 8, the entropy computation means 9, and the comparative assessment means 10 are integrated as shown in FIG. 1, the present example shows a case in which the atrial fibrillation detection system 1 has a system configuration (device configuration) comprising, as shown in FIG. 3: a small and lightweight pulse interval measurement sensor 2 for measuring pulse intervals, the pulse interval measurement means 4 being provided to the pulse interval measurement sensor 2; and an analyzer 3 in which the pulse interval conversion means 8, the entropy computation means 9, and the comparative assessment means 10 are provided.

Specifically, the present example is a configuration in which the pulse interval measurement means 4 can be worn by a subject, where the pulse interval measurement sensor 2, which is configured as a small and lightweight sensor for measuring pulse intervals and which comprises the pulse interval measurement means 4, is worn by the subject, making it possible to assess whether atrial fibrillation is occurring without hindering daily activities.

In the present example, the analyzer 3 may be configured so that the subject can carry the analyzer 3 around, or may be configured so as to be remotely installable.

In the latter case, it is preferable to adopt a configuration in which, for example, the pulse interval measurement sensor 2 and the analyzer 3 are connected wirelessly or by a communication line.

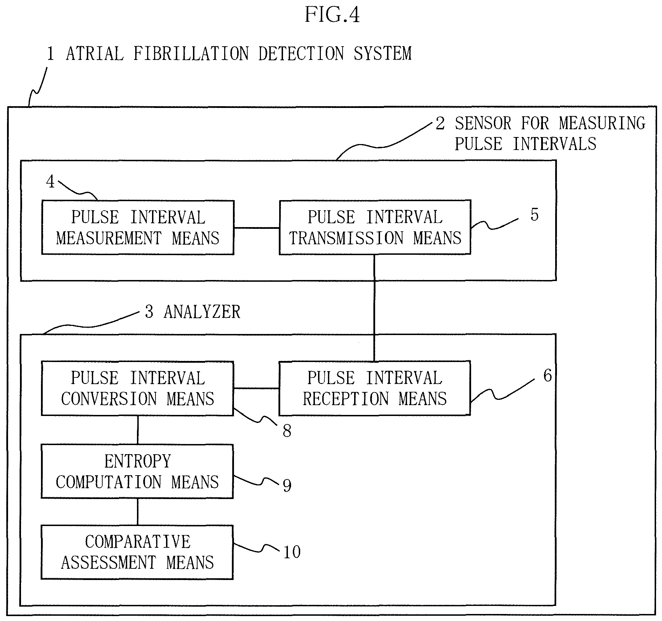

Specifically, as shown in FIG. 4, for example, a configuration can be adopted in which pulse interval transmission means 5 is provided to the pulse interval measurement sensor 2, pulse interval reception means 6 is provided to the analyzer 3, and transmission/reception of measurement data between the pulse interval transmission means 5 and the pulse interval reception means 6 is performed. For example, in cases where the analyzer 3 is a smartphone, the pulse interval transmission means 5 and the pulse interval reception means 6 can be configured so as to use wireless communication such as Bluetooth® or WiFi, and employing such a configuration makes it possible to constantly monitor the physical state of the subject. The analyzer 3 may also be configured so as to be provided to a server on the Internet. In this case, the pulse intervals R of the subject can be transmitted to the server via the smartphone, and, for example, a physician at a distant location can recognize the pulse intervals R of the subject in real time.

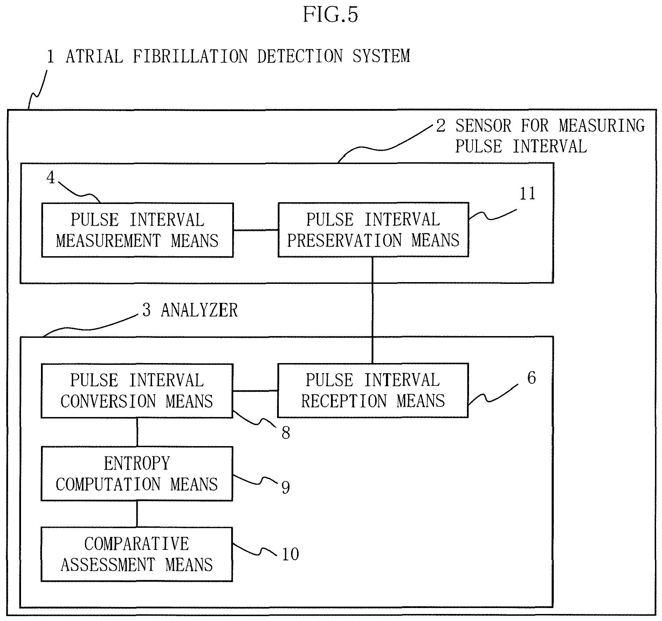

In the present example, pulse interval preservation means 11 for preserving the pulse intervals R may be provided to the pulse interval measurement sensor 2 in lieu of the pulse interval transmission means 5, as shown in FIG. 5, and a configuration may be adopted in which, after measurement by the pulse interval measurement means 4 is complete, the analyzer 3 reads the pulse intervals R from the pulse interval preservation means 11 via the pulse interval reception means 6.

Adopting such a configuration obviates the need for the subject to attend to the smartphone or to the wireless state, and makes it possible to carry out activities much closer to an everyday routine, even during measurement. In addition, if the subject is given only the pulse interval measurement sensor 2, the pulse interval measurement sensor 2 is returned after measurement to, *inter alia,* an analysis center at which the analyzer 3 is provided, the pulse intervals R preserved by the pulse interval preservation means 11 provided to the pulse interval measurement sensor 2 are read out at the analysis center, and an assessment is made as to whether atrial fibrillation is occurring in the subject, then the present invention can also be adapted to diagnostic tests, etc.

The present invention is not limited to examples 1 through 3; the specific configuration of the structural elements can be designed as appropriate.

**Claims**

1. An atrial fibrillation detection system that detects whether atrial fibrillation is occurring in a subject, the atrial fibrillation detection system being **characterized in** comprising:

   pulse interval measurement means that measures the pulse intervals of a heart; pulse interval conversion means that performs conversion, using a prescribed function, so that the extent of variation in the pulse intervals R obtained by the pulse interval measurement means is substantially fixed; entropy computation means that calculates entropy S from pulse interval images r obtained through conversion by the pulse interval conversion means; and
   comparative assessment means that compares the entropy S calculated by the entropy computation means and a prescribed threshold value, and that, in cases where the entropy S is greater than the threshold value, assesses that atrial fibrillation is occurring.

2. The atrial fibrillation detection system according to claim 1, **characterized in that** the pulse interval conversion means is configured so as to convert the pulse intervals R to the pulse interval images r using formula (1).
   [Mathematical formula 1]

$$r = f(R) = \alpha \log R + \beta R + \gamma \qquad \cdots (1)$$

   In the formula, $\alpha$, $\beta$, and $\gamma$ are constants.

3. The atrial fibrillation detection system according to either one of claims 1 and 2, **characterized in that** the entropy computation means is configured so as to calculate the entropy S as described below:
   A pulse interval image space of the pulse interval conversion means is divided into a prescribed number M of segments, and the entropy S is calculated from the N pulse interval images r obtained by the pulse interval conversion means according to formula.
   [Mathematical formula 2]

$$S = \log N! - \sum_{m=1}^{M} \log n_m! \qquad \cdots (2)$$

In the formula, $n_m$ refers to the number of pulse interval images r included in an $m^{th}$ segment.

4. The atrial fibrillation detection system according to either one of claims 1 and 2, **characterized in that** the entropy computation means is configured so as to calculate the entropy S as described below:
A pulse interval image space of the pulse interval conversion means is divided into a prescribed number M of segments, and the entropy S is calculated from the N pulse interval images r obtained by the pulse interval conversion means according to formula (3). [Mathematical formula 3]

$$S = -\sum_{m=1}^{M} \frac{n_m}{N} \log \frac{n_m}{N} \qquad \cdots (3)$$

In the formula, $n_m$ refers to the number of pulse interval images r included in an $m^{th}$ segment.

5. The atrial fibrillation detection system according to either one of claims 1 and 2, **characterized in that** the entropy computation means is configured so as to calculate the entropy S as described below:
Prescribed distributions g are kept centered on the values of each of the N pulse interval images r obtained by the pulse interval conversion means, a distribution G obtained from the sum of the N distributions g is normalized using formula (4), a probability density distribution p is calculated using formula (5), and the entropy S is calculated using formula (6).
[Mathematical formula 4]

$$G(r) = \sum_{n=1}^{N} g(r, r_n) \qquad \cdots (4)$$

[Mathematical formula 5]

$$p(r) = \frac{G(r)}{\int_{r_1}^{r_2} G(r)dr} \qquad \cdots (5)$$

[Mathematical formula 6]

$$S = -\int_{r1}^{r2} p(r) \log p(r) \, dr \qquad \cdots (6)$$

In the formulas, r is a variable in the pulse interval image space of the pulse interval conversion means, and $r_1$ and $r_2$ are the lower end and upper end of a prescribed integral segment.

6. The atrial fibrillation detection system according to claim 5, **characterized in that** the distribution g is represented by formula (7).
[Mathematical formula 7]

$$g(r, r_n) = c \exp \left\{ -\frac{(r - r_n)^2}{2\sigma^2} \right\} \qquad \cdots (7)$$

In the formula, r is a variable in the pulse interval image space of the pulse interval conversion means, and $r_n$ is a pulse interval image r converted by the pulse interval conversion means. In addition, c is an arbitrary constant other than 0, and $\sigma$ is a standard deviation representing spreading of the distribution.

7. The atrial fibrillation detection system according to any one of claims 1 to 6, **characterized in** being configured so that: the pulse interval measurement means is provided to a pulse interval measurement sensor, and the pulse interval conversion means, the entropy computation means, and the comparative assessment means are provided to an analyzer.

8. The atrial fibrillation detection system according to claim 7, **characterized in** being configured so that: the pulse interval measurement sensor has pulse interval transmission means that transmits the pulse intervals R to the analyzer, or a pulse interval preservation means that preserves the pulse intervals R; and the pulse intervals R are inputted to the analyzer via the pulse interval transmission means or the pulse interval preservation means provided to the pulse interval measurement sensor, whereby atrial fibrillation is detected.

9. The atrial fibrillation detection system according to any one of claims 1 to 8, **characterized in** comprising extrasystole exclusion means that excludes pulse intervals derived from extrasystoles from among the pulse intervals R measured by the pulse interval measurement means, and being configured so that the entropy S is calculated by the entropy computation means from the pulse interval images r obtained through conversion by the pulse interval conversion means so that the extent of variation in the pulse intervals R, from which pulse intervals derived from extrasystoles have been excluded by the extrasystole exclusion means, is substantially fixed.

10. The atrial fibrillation detection system according to claim 9, **characterized in that** the extrasystole exclusion means is configured so as to exclude pulse intervals derived from extrasystoles on the basis of the magnitude of the difference between the pulse intervals R measured by the pulse interval measurement means and the average value of the pulse intervals R.

11. The atrial fibrillation detection system according to claim 10, **characterized in that** the extrasystole exclusion means is configured so as to exclude pulse intervals derived from extrasystoles as described below:
Pulse intervals $R_n$ and $R_{n+1}$ that satisfy formulas (9) through (11) are excluded, where $R_n$ is a time series of pulse intervals R measured by the pulse interval measurement means, formula (8) indicates a normalized pulse interval $NDR_n$, and $R_n$ is the average value calculated using a prescribed method.
[Mathematical formula 8]

$$NDR_n = \frac{2(R_n - R_{n+1})}{R_n + R_{n+1}} \qquad \cdots (8)$$

In the formula, n represents a time series, and refers to the past relative to n+1.
[Mathematical formula 9]

$$NDR_{n-1} > A, \qquad NDR_n < -B \qquad \cdots (9)$$

[Mathematical formula 10]

$$\frac{R_n}{\bar{R}_n} < C, \qquad \frac{R_{n+1}}{\bar{R}_{n+1}} > D, \qquad \cdots (10)$$

[Mathematical formula 11]

$$E < \frac{R_n + R_{n+1}}{\bar{\bar{R}}_n + \bar{R}_{n+1}} < F \qquad \cdots (1\,1)$$

In the formulas, each of the threshold values A, B, C, D, E, and F is a value greater than 0, and E < F.

# FIG.1

1 ATRIAL FIBRILLATION DETECTION SYSTEM

| | |
|---|---|
| PULSE INTERVAL MEASUREMENT MEANS | 4 |
| PULSE INTERVAL CONVERSION MEANS | 8 |
| ENTROPY COMPUTATION MEANS | 9 |
| COMPARATIVE ASSESSMENT MEANS | 10 |

# FIG.2

1 ATRIAL FIBRILLATION DETECTION SYSTEM

| | |
|---|---|
| PULSE INTERVAL MEASUREMENT MEANS | 4 |
| EXTRASYSTOLE EXCLUSION MEANS | 7 |
| PULSE INTERVAL CONVERSION MEANS | 8 |
| ENTROPY COMPUTATION MEANS | 9 |
| COMPARATIVE ASSESSMENT MEANS | 10 |

FIG.3

1 ATRIAL FIBRILLATION DETECTION SYSTEM

FIG.4

1 ATRIAL FIBRILLATION DETECTION SYSTEM

2 SENSOR FOR MEASURING PULSE INTERVALS

| PULSE INTERVAL MEASUREMENT MEANS | PULSE INTERVAL TRANSMISSION MEANS |
|---|---|

4

5

3 ANALYZER

| PULSE INTERVAL CONVERSION MEANS | PULSE INTERVAL RECEPTION MEANS |
|---|---|

6

8

| ENTROPY COMPUTATION MEANS |
|---|

9

| COMPARATIVE ASSESSMENT MEANS |
|---|

10

FIG.5

1 ATRIAL FIBRILLATION DETECTION SYSTEM

2 SENSOR FOR MEASURING PULSE INTERVAL

| PULSE INTERVAL MEASUREMENT MEANS | 4 |
| PULSE INTERVAL PRESERVATION MEANS | 11 |

3 ANALYZER

| PULSE INTERVAL CONVERSION MEANS | 8 |
| PULSE INTERVAL RECEPTION MEANS | 6 |

| ENTROPY COMPUTATION MEANS | 9 |

| COMPARATIVE ASSESSMENT MEANS | 10 |

## FIG.6

## FIG.7

FIG.8

FIG.9

## FIG.10

## FIG.11

FIG.12

FIG.13

| No. | Entropy | |
|:---:|:---:|:---:|
| | Patients with atrial fibrillation | Healthy subjects |
| 1 | 32.44 | 7.19 |
| 2 | 32.44 | 0.00 |
| 3 | 25.31 | 5.35 |
| 4 | 32.67 | 12.77 |
| 5 | 31.35 | 0.00 |
| 6 | 34.24 | 8.29 |
| 7 | 31.75 | 5.35 |
| 8 | 28.20 | 10.90 |
| 9 | 32.67 | 5.35 |
| 10 | 30.84 | 0.00 |
| 11 | 29.04 | 12.22 |
| 12 | 26.81 | 0.00 |
| 13 | 29.45 | 12.22 |
| 14 | 30.14 | 0.00 |
| 15 | 31.24 | 3.04 |
| 16 | 25.64 | 0.00 |
| 17 | 34.05 | 3.04 |
| 18 | 31.35 | 5.35 |
| 19 | 33.54 | 12.77 |
| 20 | 32.44 | 9.92 |
| Average value | 30.78 | 5.69 |
| Standard deviation | 2.63 | 4.86 |

FIG.14

| No. | Entropy | |
| --- | --- | --- |
| | Patients with atrial fibrillation | Healthy subjects |
| 1 | 1.95 | 0.41 |
| 2 | 1.95 | 0.00 |
| 3 | 1.52 | 0.31 |
| 4 | 1.96 | 0.69 |
| 5 | 1.86 | 0.00 |
| 6 | 2.11 | 0.50 |
| 7 | 1.91 | 0.31 |
| 8 | 1.69 | 0.60 |
| 9 | 1.96 | 0.31 |
| 10 | 1.86 | 0.00 |
| 11 | 1.70 | 0.66 |
| 12 | 1.61 | 0.00 |
| 13 | 1.73 | 0.66 |
| 14 | 1.79 | 0.00 |
| 15 | 1.89 | 0.19 |
| 16 | 1.48 | 0.00 |
| 17 | 2.07 | 0.19 |
| 18 | 1.89 | 0.31 |
| 19 | 2.02 | 0.69 |
| 20 | 1.95 | 0.55 |
| Average value | 1.85 | 0.32 |
| Standard deviation | 0.17 | 0.26 |

FIG.15

| No. | Entropy | |
| --- | --- | --- |
| | Patients with atrial fibrillation | Healthy subjects |
| 1 | 9.02 | 7.50 |
| 2 | 8.97 | 7.34 |
| 3 | 8.61 | 7.51 |
| 4 | 9.06 | 7.64 |
| 5 | 9.09 | 7.40 |
| 6 | 9.19 | 7.57 |
| 7 | 9.12 | 7.45 |
| 8 | 8.73 | 7.48 |
| 9 | 8.95 | 7.49 |
| 10 | 8.96 | 7.33 |
| 11 | 8.81 | 7.22 |
| 12 | 8.70 | 7.40 |
| 13 | 8.89 | 7.55 |
| 14 | 8.86 | 7.26 |
| 15 | 8.91 | 7.43 |
| 16 | 8.57 | 7.26 |
| 17 | 9.14 | 7.33 |
| 18 | 8.83 | 7.47 |
| 19 | 9.06 | 7.39 |
| 20 | 9.02 | 7.37 |
| Average value | 8.92 | 7.42 |
| Standard deviation | 0.17 | 0.11 |

## FIG.16

| No. | Method in patent document 1 | | Entropy | | Entropy after exclusion of extrasystole | |
|---|---|---|---|---|---|---|
| | With extra-systole | Patient with atrial fibril-lation | With extra-systole | Patient with atrial fibril-lation | With extra-systole | Patient with atrial fibril-lation |
| 1 | No (3) | Yes (17) | 7.67 | 9.02 | 7.35 | 9.00 |
| 2 | No (3) | Yes (17) | 7.61 | 8.97 | 7.31 | 8.91 |
| 3 | No (4) | Yes (9) | 7.79 | 8.61 | 7.52 | 8.59 |
| 4 | No (2) | Yes (16) | 7.95 | 9.06 | 7.60 | 8.75 |
| 5 | No (3) | Yes (16) | 7.86 | 9.09 | 7.56 | 9.08 |
| 6 | No (5) | Yes (19) | 8.27 | 9.19 | 7.73 | 9.17 |
| 7 | No (4) | Yes (18) | 7.97 | 9.12 | 7.39 | 9.02 |
| 8 | Yes (9) | Yes (13) | 8.11 | 8.73 | 7.86 | 8.64 |
| 9 | Yes (7) | Yes (18) | 8.05 | 8.95 | 7.61 | 8.86 |
| 10 | No (6) | Yes (17) | 7.82 | 8.96 | 7.23 | 8.86 |
| 11 | Yes (9) | Yes (11) | 8.15 | 8.81 | 7.36 | 8.79 |
| 12 | Yes (12) | Yes (15) | 8.25 | 8.70 | 7.38 | 8.49 |
| 13 | Yes (8) | Yes (13) | 7.77 | 8.89 | 7.72 | 8.84 |
| 14 | Yes (11) | Yes (12) | 8.18 | 8.86 | 8.14 | 8.76 |
| 15 | Yes (8) | Yes (11) | 8.10 | 8.91 | 7.63 | 8.88 |
| 16 | Yes (11) | Yes (12) | 8.10 | 8.57 | 7.96 | 8.56 |
| 17 | Yes (11) | Yes (18) | 8.35 | 9.14 | 7.61 | 8.73 |
| 18 | Yes (11) | Yes (16) | 8.17 | 8.83 | 7.24 | 8.75 |
| 19 | Yes (12) | Yes (17) | 8.17 | 9.06 | 7.37 | 8.88 |
| 20 | Yes (15) | Yes (16) | 8.19 | 9.02 | 7.18 | 8.87 |
| Average value | | | 8.03 | 8.92 | 7.54 | 8.82 |
| Standard Deviation | | | 0.21 | 0.17 | 0.26 | 0.17 |

**EP 3 838 150 A1**

## EUROPEAN SEARCH REPORT

Europäisches Patentamt
European Patent Office
Office européen des brevets

Application Number

EP 20 21 2430

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/107266 A1 (ABOA LEGIS OY [FI]) 23 July 2015 (2015-07-23) * page 2, lines 23, 24 * * page 3, line 35 * * page 5, lines 3-6, 8-12, 18, 21, 22 * * page 6, lines 1-5, 8-10 * * figures 1, 3 * | 1-11 | INV. A61B5/361 A61B5/00 A61B5/364 A61B5/024 |
| A | LLAMEDO M ET AL: "An Automatic Patient-Adapted ECG Heartbeat Classifier Allowing Expert Assistance", IEEE TRANSACTIONS ON BIOMEDICAL ENGINEERING, IEEE SERVICE CENTER, PISCATAWAY, NJ, USA, vol. 59, no. 8, 1 August 2012 (2012-08-01), pages 2312-2320, XP011490166, ISSN: 0018-9294, DOI: 10.1109/TBME.2012.2202662 * tables 2, 3 * | 2 | |
| A | DEMAZUMDER DEEPTANKAR ET AL: "Dynamic Analysis of Cardiac Rhythms for Discriminating Atrial Fibrillation From Lethal Ventricular Arrhythmias", CIRCULATION: ARRHYTHMIA AND ELECTROPHYSIOLOGY, vol. 6, no. 3, 1 June 2013 (2013-06-01), pages 555-561, XP055801456, United States ISSN: 1941-3149, DOI: 10.1161/CIRCEP.113.000034 * abstract * | 3 | **TECHNICAL FIELDS SEARCHED (IPC)** A61B |

-/--

The present search report has been drawn up for all claims

| Place of search | Date of completion of the search | Examiner |
|---|---|---|
| The Hague | 4 May 2021 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 21 2430

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XIAOLIN ZHOU ET AL: "Automatic online detection of atrial fibrillation based on symbolic dynamics and Shannon entropy", BIOMEDICAL ENGINEERING ONLINE, BIOMED CENTRAL LTD, LONDON, GB, vol. 13, no. 1, 17 February 2014 (2014-02-17), page 18, XP021180868, ISSN: 1475-925X, DOI: 10.1186/1475-925X-13-18 * equation (6) * | 4 | |
| A | MICHALOWICZ JOSEPH ET AL: "Calculation of Differential Entropy for a Mixed Gaussian Distribution", ENTROPY, vol. 10, no. 3, 25 August 2008 (2008-08-25), pages 200-206, XP055801484, DOI: 10.3390/entropy-e10030200 * abstract * | 5,6 | |
| A | AKTARUZZAMAN M ET AL: "Sample Entropy parametric estimation for heart rate variability analysis", COMPUTING IN CARDIOLOGY, N/A, 22 September 2013 (2013-09-22), pages 429-432, XP032550480, ISSN: 2325-8861 ISBN: 978-1-4799-0884-4 [retrieved on 2014-01-15] * page 429, right-hand column, lines 10-12 * | 5,6 | |

TECHNICAL FIELDS
SEARCHED (IPC)

-/--

The present search report has been drawn up for all claims

| Place of search | Date of completion of the search | Examiner |
|---|---|---|
| The Hague | 4 May 2021 | Meyer, Wolfgang |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 3

Europäisches
Patentamt

European
Patent Office

Office européen
des brevets

## EUROPEAN SEARCH REPORT

Application Number

EP 20 21 2430

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ATANASOSKI V ET AL: "Unsupervised Classification of Premature Ventricular Contractions Based on RR Interval and Heartbeat Morphology", 2018 14TH SYMPOSIUM ON NEURAL NETWORKS AND APPLICATIONS (NEUREL), IEEE, 20 November 2018 (2018-11-20), pages 1-6, XP033483489, DOI: 10.1109/NEUREL.2018.8586997 [retrieved on 2018-12-21] * page 3, left-hand column, lines 1-28 * ----- | 9-11 | |

TECHNICAL FIELDS
SEARCHED      (IPC)

The present search report has been drawn up for all claims

| Place of search | Date of completion of the search | Examiner |
|---|---|---|
| The Hague | 4 May 2021 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 2430

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2021

| Patent document cited in search report | Publication date | Patent family member(s) | | Publication date |
|---|---|---|---|---|
| WO 2015107266 A1 | 23-07-2015 | EP | 3094244 A1 | 23-11-2016 |
| | | JP | 6263635 B2 | 17-01-2018 |
| | | JP | 2017504414 A | 09-02-2017 |
| | | MX | 365101 B | 22-05-2019 |
| | | WO | 2015107266 A1 | 23-07-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6150825 B